# EUROPEAN PATENT SPECIFICATION

(11) **EP 0 832 233 B1**
(45) Date of publication and mention of the grant of the patent: **06.10.2004**
(21) Application number: 95923767.8
(22) Date of filing: 06.06.1995
(51) Int. Cl.: C12N 15/19, C12N 15/63, C12N 5/10, C12N 1/19, C12N 1/21, C07K 14/52, A61K 38/19

(54) **HUMAN CHEMOKINE BETA-13**
HUMANES CHEMOKIN BETA-13
CHEMOKINE BETA-13 HUMAINE

(43) Date of publication of application: 01.04.1998
(73) Proprietor: HUMAN GENOME SCIENCES, INC., Rockville, MD 20850-3338 (US); SMITHKLINE BEECHAM CORPORATION, King of Prussia, PA 19406-0939 (US)
(72) Inventor: LI, Haodong, Gaithersburg, MD 20878 (US); SEIBEL, George, Saint Davids, PA 19087 (US)
(74) Representative: VOSSIUS & PARTNER
(86) International application number: PCT/US1995/007294
(87) International publication number: WO 1996/039521

(56) References cited:
- WO-A-90/07009
- MILLER M.D. ET AL.: "Biology and biochemistry of the chemokines: a family of chemotactic and inflammatory cytokines" CRITICAL REVIEWS IN IMMUNOLOGY, vol. 12, no. 1/02, 1992, pages 17-46, XP002050850
- CYTOKINE, Volume 3, Number 3, issued May 1991, T.J. SCALL, "Biology of the RANTES/SIS Cytokine Family", pages 165-183.

### SEQUENCE LISTING

(1) GENERAL INFORMATION:
   (i) APPLICANT: LI, ET AL.
   (ii) TITLE OF INVENTION: HUMAN CHEMOKINE BETA 13
   (iii) NUMBER OF SEQUENCES: 8
   (iv) CORRESPONDENCE ADDRESS:
      (A) ADDRESSEE: HUMAN GENOME SCIENCES, INC.
      (B) STREET: 9410 KEY WEST AVENUE
      (C) CITY: ROCKVILLE,
      (D) STATE: MD
      (E) COUNTRY: 20850
      (F) ZIP: US
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30
   (vi) CURRENT APPLICATION DATA:
      (A) APPLICATION NUMBER: 95 92 3767.8-
      (B) FILING DATE: 06 June 1995
      (C) CLASSIFICATION:
   (viii) ATTORNEY/AGENT INFORMATION:
      (A) NAME: Vossius & Partner
      (B) REGISTRATION NUMBER:
      (C) REFERENCE/DOCKET NUMBER: B2960 EPS3
   (ix) TELECOMMUNICATION INFORMATION:
      (A) TELEPHONE:
      (B) TELEFAX:
(2) INFORMATION FOR SEQ ID NO:1:
   (i) SEQUENCE CHARACTERISTICS:
      (A) LENGTH: 282 base pairs
      (B) TYPE: nucleic acid
      (C) STRANDEDNESS: single
      (D) TOPOLOGY: linear
   (ii) MOLECULE TYPE: cDNA
   (xi) SEQUENCE DESCRIPTION: SEQ ID NO:1:
   ATGGCTCGCC TACAGACTGC ACTCCTGGTT GTCCTCGTCC TCCTTGCTGT GGCGCTTCAA     60
   GCAACTGAGG CAGGCCCCTA CGGCGCCAAC ATGGAAGACA GCGTCTGCTG CCGTGATTAC    120
   GTCCGTTACC GTCTGCCCCT GCGCGTGGTG AAACACTTCT ACTGGACCTC AGACTCCTGC    180
   CCGAGGCCTG GCGTGGTGTT GCTAACCTTC AGGGATAAGG AGATCTGTGC CGATCCCAGA    240
   GTGCCCTGGG TGAAGATGAT TCTCAATAAG CTGAGCCAAT GA                       282
(2) INFORMATION FOR SEQ ID NO:2:
   (i) SEQUENCE CHARACTERISTICS:
      (A) LENGTH: 93 amino acids
      (B) TYPE: amino acid
      (C) STRANDEDNESS:
      (D) TOPOLOGY: linear
   (ii) MOLECULE TYPE: protein
   (xi) SEQUENCE DESCRIPTION: SEQ ID NO:2:
   Met Ala Arg Leu Gln Thr Ala Leu Leu Val Val Leu Val Leu Leu
               -25                 -20                 -15
   Ala Val Ala Leu Gln Ala Thr Glu Ala Gly Pro Tyr Gly Ala Asn
               -10                  -5                   1
   Met Glu Asp Ser Val Cys Cys Arg Asp Tyr Val Arg Tyr Arg Leu
             5                  10                  15
   Pro Leu Arg Val Val Lys His Phe Tyr Trp Thr Ser Asp Ser Cys
            20                  25                  30
   Pro Arg Pro Gly Val Val Leu Leu Thr Phe Arg Asp Lys Glu Ile
            35                  40                  45
   Cys Ala Asp Pro Arg Val Pro Trp Val Lys Met Ile Leu Asn Lys
            50                  55                  60
   Leu Ser Gln
            65
(2) INFORMATION FOR SEQ ID NO:3:
   (i) SEQUENCE CHARACTERISTICS:
      (A) LENGTH: 25 base pairs
      (B) TYPE: nucleic acid
      (C) STRANDEDNESS: single
      (D) TOPOLOGY: linear
   (ii) MOLECULE TYPE: oligonucleotide
   (xi) SEQUENCE DESCRIPTION: SEQ ID NO:3:
   CCCGCATGCC CAACATGGAA GACAG                                      25
(2) INFORMATION FOR SEQ ID NO:4:
   (i) SEQUENCE CHARACTERISTICS:
      (A) LENGTH: 27 base pairs
      (B) TYPE: nucleic acid
      (C) STRANDEDNESS: single
      (D) TOPOLOGY: linear
   (ii) MOLECULE TYPE: Oligonucleotide
   (xi) SEQUENCE DESCRIPTION: SEQ ID NO:4:
   AAAGGATCCT TGGCTCAGCT TATTGAG 27
(2) INFORMATION FOR SEQ ID NO:5:
   (i) SEQUENCE CHARACTERISTICS:
      (A) LENGTH: 31 base pairs
      (B) TYPE: nucleic acid
      (C) STRANDEDNESS: single
      (D) TOPOLOGY: linear
   (ii) MOLECULE TYPE: Oligonucleotide
   (xi) SEQUENCE DESCRIPTION: SEQ ID NO:5:
   AAAAAGCTTA ACATAGGCTC GCCTACAGAC T 31
(2) INFORMATION FOR SEQ ID NO:6:
   (i) SEQUENCE CHARACTERISTICS:
      (A) LENGTH: 60 base pairs
      (B) TYPE: nucleic acid
      (C) STRANDEDNESS: single
      (D) TOPOLOGY: linear
   (ii) MOLECULE TYPE: Oligonucleotide
   (xi) SEQUENCE DESCRIPTION: SEQ ID NO:6:
   CGCTCTAGAT TAAGCGTAGT CTGGGACGTC GTATGGGTAT TGGCTCAGCT TATTGAGAAT 60
(2) INFORMATION FOR SEQ ID NO:7:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: Oligonucleotide
   (xi) SEQUENCE DESCRIPTION: SEQ ID NO:7:
   AAAGGATCCG CCACCATGGC TCGCCTACAG ACT 33
(2) INFORMATION FOR SEQ ID NO:8:
   (i) SEQUENCE CHARACTERISTICS:
      (A) LENGTH: 26 base pairs
      (B) TYPE: nucleic acid
      (C) STRANDEDNESS: single
      (D) TOPOLOGY: linear
   (ii) MOLECULE TYPE: Oligonucleotide
   (xi) SEQUENCE DESCRIPTION: SEQ ID NO:8:

## Description

This invention relates to newly identified polynucleotides, polypeptides encoded by such polynucleotides, the use of such polynucleotides and polypeptides, as well as the production of such polynucleotides and polypeptides. More particularly, the polypeptide of the present invention has been putatively identified as a human chemokine polypeptides, sometimes hereinafter referred to as human chemokine beta-13 (Ckβ-13). The invention also relates to inhibiting the action of such polypeptides.

Chemokines, also referred to as intercrine cytokines, are a subfamily of structurally and functionally related cytokines. These molecules are 8-10 kd in size. In general, chemokines exhibit 20% to 75% homology at the amino acid level and are characterized by four conserved cysteine residues that form two disulfide bonds. Based on the arrangement of the first two cysteine residues, chemokines have been classified into two subfamilies, alpha and beta. In the alpha subfamily, the first two cysteines are separated by one amino acid and hence are referred to as the "C-X-C" subfamily. In the beta subfamily, the two cysteines are in an adjacent position and are, therefore, referred to as the "C-C" subfamily. Thus far, at least nine different members of this family have been identified in humans.

The intercrine cytokines exhibit a wide variety of functions. A hallmark feature is their ability to elicit chemotactic migration of distinct cell types, including monocytes, neutrophils, T lymphocytes, basophils and fibroblasts. Many chemokines have proinflammatory activity and are involved in multiple steps during an inflammatory reaction. These activities include stimulation of histamine release, lysosomal enzyme and leukotriene release, increased adherence of target immune cells to endothelial cells, enhanced binding of complement proteins, induced expression of granulocyte adhesion molecules and complement receptors, and respiratory burst. In addition to their involvement in inflammation, certain chemokines have been shown to exhibit other activities. For example, macrophage inflammatory protein 1 (MIP-1) is able to suppress hematopoietic stem cell proliferation, platelet factor-4 (PF-4) is a potent inhibitor of endothelial cell growth, Interleukin-8 (IL-8) promotes proliferation of keratinocytes, and GRO is an autocrine growth factor for melanoma cells.

In light of the diverse biological activities, it is not surprising that chemokines have been implicated in a number of physiological and disease conditions, including lymphocyte trafficking, wound healing, hematopoietic regulation and immunological disorders such as allergy, asthma and arthritis.

Members of the "C-C" branch exert their effects on the following cells: eosinophils which destroy parasites to lessen parasitic infection and cause chronic inflammation in the airways of the respiratory system; monocytes and macrophages which suppress tumor formation in vertebrates; T lymphocytes which attract T cells and basophils which release histamine which plays a role in allergic inflammation.

While members of the C-C branch act predominantly on mononuclear cells and members of the C-X-C branch act predominantly on neutrophils a distinct chemoattractant property cannot be assigned to a chemokine based on this guideline. Some chemokines from one family show characteristics of the other.

The polypeptide of the present invention has the conserved cysteine "C-C" region, and has amino acid sequence homology to known chemokines.

In accordance with one aspect of the present invention, there are provided novel polypeptides as well as biologically active and diagnostically or therapeutically useful fragments, analogs and derivatives thereof.

In accordance with another aspect of the present invention, there are provided isolated nucleic acid molecules encoding such polypeptides, including mRNAs, DNAs, cDNAs, genomic DNA as well as biologically active and diagnostically or therapeutically useful fragments, analogs and derivatives thereof.

In accordance with another aspect of the present invention there are provided nucleic acid probes comprising nucleic acid molecules of sufficient length to specifically hybridize to nucleic acid sequences encoding the polypeptide of the present invention.

In accordance with yet a further aspect of the present invention, there is provided a process for producing such polypeptide by recombinant techniques which comprises culturing recombinant prokaryotic and/or eukaryotic host cells, containing a nucleic acid sequence encoding a polypeptide of the present invention, under conditions promoting expression of said protein and subsequent recovery of said protein.

In accordance with yet a further aspect of the present invention, there is provided a process for utilizing such polypeptides, or polynucleotides encoding such polypeptides for therapeutic purposes, for example, to treat solid tumors, chronic infections, leukemia, T-cell mediated auto-immune diseases, parasitic infections, psoriasis, to regulate hematopoiesis, to stimulate growth factor activity, to inhibit angiogenesis and to promote wound healing.

In accordance with yet a further aspect of the present invention, there are provided antibodies against such polypeptides.

In accordance with yet another aspect of the present invention, there are provided antagonists to such polypeptides, which may be used to inhibit the action of such polypeptides, for example, in the treatment of certain auto-immune diseases, atherosclerosis, chronic inflammatory and infectious diseases, histamine and IgE-mediated allergic reactions, prostaglandin-independent fever, bone marrow failure, silicosis, sarcoidosis, rheumatoid arthritis, and hyper-eosinophilic syndrome.

In accordance with another aspect of the present invention there is provided a method of diagnosing a disease or a susceptibility to a disease related to a mutation in the nucleic acid sequences of the present invention and to altered levels of the protein encoded by such nucleic acid sequences.

In accordance with yet a further aspect of the present invention, there is provided a process for utilizing such polypeptides, or polynucleotides encoding such polypeptides, for *in vitro* purposes related to scientific research, synthesis of DNA and manufacture of DNA vectors.

These and other aspects of the present invention should be apparent to those skilled in the art from the teachings herein.

The following drawings are illustrative of embodiments of the invention and are not meant to limit the scope of the invention as encompassed by the claims.

Figure 1 displays the cDNA sequence and corresponding deduced amino acid sequence of Ckβ-13. The initial 28 amino acids represent the leader sequence such that the putative mature polypeptide comprises 65 amino acids. The standard one-letter abbreviations for amino acids are used. Sequencing was performed using a 373 Automated DNA sequencer (Applied Biosystems, Inc.).

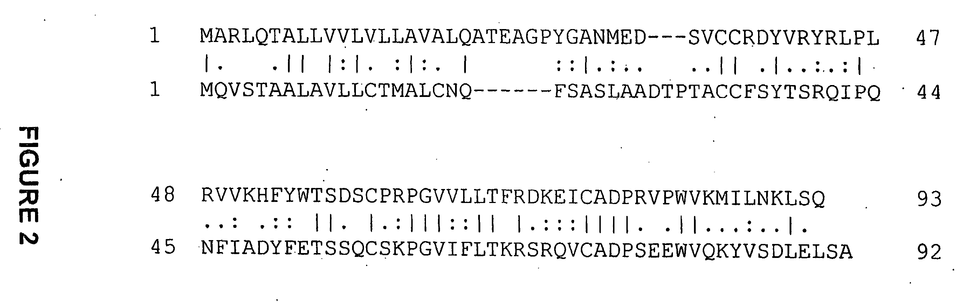

Figure 2 displays the amino acid sequence homology between Ckβ-13 (top) and human MIP-1α polypeptide (bottom).

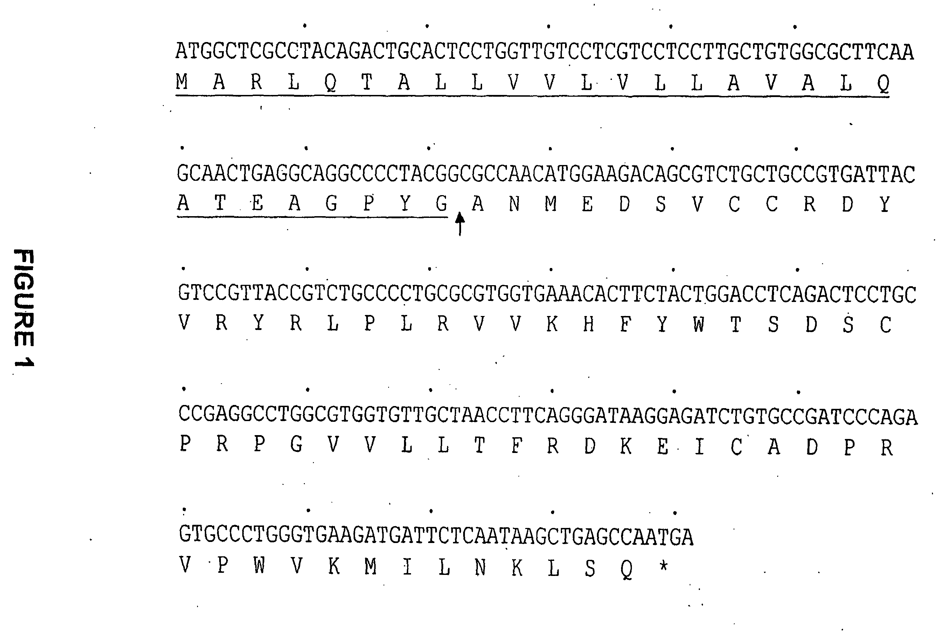

In accordance with an aspect of the present invention, there are provided isolated nucleic acids (polynucleotides) which encode for the mature polypeptide having the deduced amino acid sequence of Figure 1 (SEQ ID NO:2) or for the mature polypeptide encoded by the cDNA of the clone(s) deposited as ATCC Deposit No. 97113 on April 28, 1995.

Polynucleotides encoding Ckβ-13 have been isolated from an activated monocyte cDNA library. Ckβ-13 is a member of the C-C branch of chemokines. It contains an open reading frame encoding a protein of 93 amino acid residues of which approximately the first 28 amino acids residues are the putative leader sequence such that the mature protein comprises 65 amino acids. The protein has structural homology to chemokine polypeptides and the homology to the human MIP-1α polypeptide with 33% identity and 53% similarity over the entire sequence is used solely as an example.

The four spatially conserved cysteine residues in chemokines are found in the polypeptide of the present invention as can be seen in Figure 1.

The polynucleotides of the present invention may be in the form of RNA or in the form of DNA, which DNA includes cDNA, genomic DNA, and synthetic DNA. The DNA may be doublestranded or single-stranded, and if single stranded may be the coding strand or non-coding (anti-sense) strand. The coding sequence which encodes the mature polypeptides may be identical to the coding sequence shown in Figures 1 (SEQ ID NO:1) or that of the deposited clone(s) or may be a different coding sequence which coding sequence, as a result of the redundancy or degeneracy of the genetic code, encodes the same mature polypeptides as the DNA of Figure 1 (SEQ ID NO:1) or the deposited cDNA.

The polynucleotides which encode for the mature polypeptide of Figure 1 (SEQ ID NO:2) or for the mature polypeptide encoded by the deposited cDNA may include: only the coding sequence for the mature polypeptide; the coding sequence for the mature polype de and additional coding sequence such as a leader or secretory sequence or a proprotein sequence; the coding sequence for the mature polypeptide (and optionally additional coding sequence) and non-coding sequence, such as introns or non-coding sequence 5' and/or 3' of the coding sequence for the mature polypeptides.

Thus, the term "polynucleotide encoding a polypeptide" encompasses a polynucleotide which includes only coding sequence for the polypeptide as well as a polynucleotide which includes additional coding and/or non-coding sequence.

The present invention further relates to variants of the hereinabove described polynucleotides which encode for fragments, analogs and derivatives of the polypeptide having the deduced amino acid sequence of Figure 1 (SEQ ID NO:2) or the polypeptide encoded by the cDNA of the deposited clone(s). The variants of the polynucleotides may be a naturally occurring allelic variant of the polynucleotides or a non-naturally occurring variant of the polynucleotides.

Thus, the present invention includes polynucleotides encoding the same mature polypeptide as shown in Figures 1 (SEQ ID NO:2) or the same mature polypeptide encoded by the cDNA of the deposited clone(s) as well as variants of such polynucleotides which variants encode for a fragment, derivative or analog of the polypeptide of Figure 1 (SEQ ID NO:2) or the polypeptide encoded by the cDNA of the deposited clone(s). Such nucleotide variants include deletion variants, substitution variants and addition or insertion variants.

As hereinabove indicated, the polynucleotides may have a coding sequence which is a naturally occurring allelic variant of the coding sequence shown in Figure 1 (SEQ ID NO:1) or of the coding sequence of the deposited clone(s). As known in the art, an allelic variant is an alternate form of a polynucleotide sequence which may have a substitution, deletion or addition of one or more nucleotides, which does not substantially alter the function of the encoded polypeptide.

The present invention also includes polynucleotides, wherein the coding sequence for the mature polypeptidesmay be fused in the same reading frame to a polynucleotide sequence which aids in expression and secretion of a polypeptide from a host cell, for example, a leader sequence which functions as a secretory sequence for controlling transport of a polypeptide from the cell. The polypeptide having a leader sequence is a preprotein and may have the leader sequence cleaved by the host cell to form the mature form of the polypeptide. The polynucleotides may also encode for a proprotein which is the mature protein plus additional 5' amino acid residues. A mature protein having a prosequence is a proprotein and is an inactive form of the protein. Once the prosequence is cleaved an active mature protein remains.

Thus, for example, the polynucleotides of the present invention may encode for a mature protein, or for a protein having a prosequence or for a protein having both a prosequence and a presequence (leader sequence).

The polynucleotides of the present invention may also have the coding sequence fused in frame to a marker sequence which allows for purification of the polypeptide of the present invention. The marker sequence may be a hexahistidine tag supplied by a pQE-9 vector to provide for purification of the mature polypeptide fused to the marker in the case of a bacterial host, or, for example, the marker sequence may be a hemagglutinin (HA) tag when a mammalian host, e.g. COS-7 cells, is used. The HA tag corresponds to an epitope derived from the influenza hemagglutinin protein (Wilson, I., et al., Cell, 37:767 (1984)).

The term "gene" means the segment of DNA involved in producing a polypeptide chain; it includes regions preceding and following the coding region (leader and trailer) as well as intervening sequences (introns) between individual coding segments (exons).

Fragments of the full length Ckβ-13 gene may be used as a hybridization probe for a cDNA library to isolate the full length gene and to isolate other genes which have a high sequence similarity to the gene or similar biological activity. Probes of this type preferably have at least 30 bases and may contain, for example, 50 or more bases. The probe may also be used to identify a cDNA clone corresponding to a full length transcript and a genomic clone or clones that contain the complete Ckβ-13 gene including regulatory and promotor regions, exons, and introns. An example of a screen comprises isolating the coding region of the Ckβ-13 gene by using the known DNA sequence to synthesize an oligonucleotide probe. Labeled oligonucleotides having a sequence complementary to that of the gene of the present invention are used to screen a library of human cDNA, genomic DNA or mRNA to determine which members of the library the probe hybridizes to.

The present invention further relates to polynucleotides which hybridize to the hereinabove-described sequences if there is at least 70%, preferably at least 90%, and more preferably at least 95% identity between the sequences. The present invention particularly relates to polynucleotides which hybridize under stringent conditions to the hereinabove-described polynucleotides. As herein used, the term "stringent conditions" means hybridization will occur only if there is at least 95% and preferably at least 97% identity between the sequences. The polynucleotides which hybridize to the hereinabove described polynucleotides in a preferred embodiment encode polypeptides which either retain substantially the same biological function or activity as the mature polypeptide encoded by the cDNA of Figure 1 (SEQ ID NO:1) or the deposited cDNA(s), i.e. function as a chemokine polypeptide.

Alternatively, the polynucleotides may be polynucleotides which have at least 20 bases, preferably 30 bases and more preferably at least 50 bases which hybridize to a polynucleotide of the present invention and which have an identity thereto, as hereinabove described, and which may or may not retain activity. For example, such polynucleotides may be employed as probes for the polynucleotide of SEQ ID NO:1, or for variants thereof, for example, for recovery of the polynucleotide or as a diagnostic probe or as a PCR primer.

Thus, the present invention is directed to polynucleotides having at least a 70% identity, preferably at least 90% and more preferably at least a 95% identity to a polynucleotide which encodes the polypeptide of SEQ ID NO:2 as well as fragments thereof, which fragments have at least 30 bases and preferably at least 50 bases and to polypeptides encoded by such polynucleotides.

The deposit(s) referred to herein will be maintained under the terms of the Budapest Treaty on the International Recognition of the Deposit of Micro-organisms for purposes of Patent Procedure. These deposits are provided merely as convenience to those of skill in the art and are not an admission that a deposit is required under 35 U.S.C. §112. The sequence of the polynucleotides contained in the deposited materials, as well as the amino acid sequence of the polypeptides encoded thereby, are incorporated herein by reference and are controlling in the event of any conflict with any description of sequences herein. A license may be required to make, use or sell the deposited materials, and no such license is hereby granted.

The present invention further relates to polypeptides which have the deduced amino acid sequence of Figure 1 (SEQ ID NO:2 ) or which have the amino acid sequence encoded by the deposited cDNA, as well as fragments, analogs and derivatives of such polypeptides.

The terms "fragment," "derivative" and "analog" when referring to the polypeptide of Figure 1 (SEQ ID NO:2) or that encoded by the deposited cDNA, means a polypeptide which retain essentially the same biological function or activity as such polypeptide. Thus, an analog includes a proprotein which can be activated by cleavage of the proprotein portion to produce an active mature polypeptide. A derivative or fragment may include, for example, a splice variant which has less amino acid residues than the polypeptide of Figure 1 but which still retains biological activity characteristic of human chemokine polypeptides.

The polypeptides of the present invention may be recombinant polypeptides, natural polypeptides or synthetic polypeptides, preferably recombinant polypeptides.

The fragment, derivative or analog of the polypeptide of Figure 1 (SEQ ID NO:2) or that encoded by the deposited cDNAs may be (i) one in which one or more of the amino acid residues are substituted with a conserved or non-conserved amino acid residue (preferably a conserved amino acid residue) and such substituted amino acid residue may or may not be one encoded by the genetic code, or (ii) one in which one or more of the amino acid residues includes a substituent group, or (iii) one in which the mature polypeptide is fused with another compound, such as a compound to increase the half-life of the polypeptide (for example, polyethylene glycol), or (iv) one in which the additional amino acids are fused to the mature polypeptide, such as a leader or secretory sequence or a sequence which is employed for purification of the mature polypeptide or a proprotein sequence. Such fragments, derivatives and analogs are deemed to be within the scope of those skilled in the art from the teachings herein.

The polypeptides and polynucleotides of the present invention are preferably provided in an isolated form, and preferably are purified to homogeneity.

The term "isolated" means that the material is removed from its original environment (e.g., the natural environment if it is naturally occurring). For example, a naturally-occurring polynucleotide or polypeptide present in a living animal is not isolated, but the same polynucleotide or polypeptide, separated from some or all of the coexisting materials in the natural system, is isolated. Such polynucleotides could be part of a vector and/or such polynucleotides or polypeptides could be part of a composition, and still be isolated in that such vector or composition is not part of its natural environment.

The polypeptides of the present invention include the polypeptide of SEQ ID NO:2 (in particular the mature polypeptide) as well as polypeptides which have at least 70% similarity (preferably at least 70% identity) to the polypeptide of SEQ ID NO:2 and more preferably at least 90% similarity (more preferably at least 90% identity) to the polypeptide of SEQ ID NO:2 and still more preferably at least 95% similarity (still more preferably at least 95% identity) to the polypeptide of SEQ ID NO:2 and also include portions of such polypeptides which generally contain at least 30 amino acids and more preferably at least 50 amino acids.

As known in the art "similarity" between two polypeptides is determined by comparing the amino acid seque2nce and its conserved amino acid substitutes of one polypeptide to the sequence of a second polypeptide.

Fragments or portions of the polypeptides of the present invention may be employed for producing the corresponding full-length polypeptide by peptide synthesis; therefore, the fragments may be employed as intermediates for producing the full-length polypeptides. Fragments or portions of the polynucleotides of the present invention may be used to synthesize full-length polynucleotides of the present invention.

The present invention also relates to vectors which include polynucleotides of the present invention, host cells which are genetically engineered with vectors of the invention and the production of polypeptides of the invention by recombinant techniques.

Host cells are genetically engineered (transduced or transformed or transfected) with the vectors of this invention which may be, for example, a cloning vector or an expression vector. The vector may be, for example, in the form of a plasmid, a viral particle, a phage, etc. The engineered host cells can be cultured in conventional nutrient media modified as appropriate for activating promoters, selecting transformants or amplifying the Ckβ-13 genes. The culture conditions, such as temperature, pH and the like, are those previously used with the host cell selected for expression, and will be apparent to the ordinarily skilled artisan.

The polynucleotides of the present invention may be employed for producing polypeptides by recombinant techniques. Thus, for example, the polynucleotide may be included in any one of a variety of expression vectors for expressing a polypeptide. Such vectors include chromosomal, nonchromosomal and synthetic DNA sequences, e.g., derivatives of SV40; bacterial plasmids; phage DNA; baculovirus; yeast plasmids; vectors derived from combinations of plasmids and phage DNA, viral DNA such as vaccinia, adenovirus, fowl pox virus, and pseudorabies. However, any other vector may be used as long as it is replicable and viable in the host.

The appropriate DNA sequence may be inserted into the vector by a variety of procedures. In general, the DNA sequence is inserted into an appropriate restriction endonuclease site(s) by procedures known in the art. Such procedures and others are deemed to be within the scope of those skilled in the art.

The DNA sequence in the expression vector is operatively linked to an appropriate expression control sequence(s) (promoter) to direct mRNA synthesis. As representative examples of such promoters, there may be mentioned: LTR or SV40 promoter, the E. coli. lac or trp, the phage lambda P_{L} promoter and other promoters known to control expression of genes in prokaryotic or eukaryotic cells or their viruses. The expression vector also contains a ribosome binding site for translation initiation and a transcription terminator. The vector may also include appropriate sequences for amplifying expression.

In addition, the expression vectors preferably contain one or more selectable marker genes to provide a phenotypic trait for selection of transformed host cells such as dihydrofolate reductase or neomycin resistance for eukaryotic cell culture, or such as tetracycline or ampicillin resistance in E. coli.

The vector containing the appropriate DNA sequence as hereinabove described, as well as an appropriate promoter or control sequence, may be employed to transform an appropriate host to permit the host to express the protein.

As representative examples of appropriate hosts, there may be mentioned: bacterial cells, such as E. coli, Streptomyces, Salmonella typhimurium; fungal cells, such as yeast; insect cells such as Drosophila S2 and Spodoptera Sf9; animal cells such as CHO, COS or Bowes melanoma; adenoviruses; plant cells, etc. The selection of an appropriate host is deemed to be within the scope of those skilled in the art from the teachings herein.

More particularly, the present invention also includes recombinant constructs comprising one or more of the sequences as broadly described above. The constructs comprise a vector, such as a plasmid or viral vector, into which a sequence of the invention has been inserted, in a forward or reverse orientation. In a preferred aspect of this embodiment, the construct further comprises regulatory sequences, including, for example, a promoter, operably linked to the sequence. Large numbers of suitable vectors and promoters are known to those of skill in the art, and are commercially available. The following vectors are provided by way of example. Bacterial: pQE70, pQE60, pQE-9 (Qiagen), pBS, pD10, phagescript, psiX174, pBluescript SK, pBSKS, pNH8A, pNH16a, pNH18A, pNH46A (Stratagene); pTRC99a, pKK223-3, pKK233-3, pDR540, pRIT5 (Pharmacia). Eukaryotic: pWLNEO, pSV2CAT, pOG44, pXT1, pSG (Stratagene) pSVK3, pBPV, pMSG, pSVL (Pharmacia). However, any other plasmid or vector may be used as long as they are replicable and viable in the host.

Promoter regions can be selected from any desired gene using CAT (chloramphenicol transferase) vectors or other vectors with selectable markers. Two appropriate vectors are pKK232-8 and pCM7. Particular named bacterial promoters include lacI, lacZ, T3, T7, gpt, lambda P_{R}, P_{L} and trp. Bukaryotic promoters include CMV immediate early, HSV thymidine kinase, early and late SV40, LTRs from retrovirus, and mouse metallothionein-I. Selection of the appropriate vector and promoter is well within the level of ordinary skill in the art.

In a further embodiment, the present invention relates to host cells containing the above-described constructs. The host cell can be a higher eukaryotic cell, such as a mammalian cell, or a lower eukaryotic cell, such as a yeast cell, or the host cell can be a prokaryotic cell, such as a bacterial cell. Introduction of the construct into the host cell can be effected by calcium phosphate transfection, DEAE-Dextran mediated transfection, or electroporation (Davis, L., Dibner, M., Battey, I., Basic Methods in Molecular Biology, (1986)).

The constructs in host cells can be used in a conventional manner to produce the gene products encoded by the recombinant sequences. Alternatively, the polypeptides of the invention can be synthetically produced by conventional peptide synthesizers.

Mature proteins can be expressed in mammalian cells, yeast, bacteria, or other cells under the control of appropriate promoters. Cell-free translation systems can also be employed to produce such proteins using RNAs derived from the DNA constructs of the present invention. Appropriate cloning and expression vectors for use with prokaryotic and eukaryotic hosts are described by Sambrook, et al., Molecular Cloning: A Laboratory Manual, Second Edition, Cold Spring Harbor, N.Y., (1989), the disclosure of which is hereby incorporated by reference.

Transcription of the DNA encoding the polypeptides of the present invention by higher eukaryotes is increased by inserting an enhancer sequence into the vector. Enhancers are cis-acting elements of DNA, usually about from 10 to 300 bp that act on a promoter to increase its transcription. Examples include the SV40 enhancer on the late side of the replication origin bp 100 to 270, a cytomegalovirus early promoter enhancer, the polyoma enhancer on the late side of the replication origin, and adenovirus enhancers.

Generally, recombinant expression vectors will include origins of replication and selectable markers permitting transformation of the host cell, e.g., the ampicillin resistance gene of E. coli and S. cerevisiae TRP1 gene, and a promoter derived from a highly-expressed gene to direct transcription of a downstream structural sequence. Such promoters can be derived from operons encoding glycolytic enzymes such as 3-phosphoglycerate kinase (PGK), α-factor, acid phosphatase, or heat shock proteins, among others. The heterologous structural sequence is assembled in appropriate phase with translation initiation and termination sequences, and preferably, a leader sequence capable of directing secretion of translated protein into the periplasmic space or extracellular medium. Optionally, the heterologous sequence can encode a fusion protein including an N-terminal identification peptide imparting desired characteristics, e.g., stabilization or simplified purification of expressed recombinant product.

Useful expression vectors for bacterial use are constructed by inserting a structural DNA sequence encoding a desired protein together with suitable translation initiation and termination signals in operable reading phase with a functional promoter. The vector will comprise one or more phenotypic selectable markers and an origin of replication to ensure maintenance of the vector and to, if desirable, provide amplification within the host. Suitable prokaryotic hosts for transformation include E. coli, Bacillus subtilis, Salmonella typhimurium and various species within the genera Pseudomonas, Streptomyces, and Staphylococcus, although others may also be employed as a matter of choice.

As a representative but nonlimiting example, useful expression vectors for bacterial use can comprise a selectable marker and bacterial origin of replication derived from commercially available plasmids comprising genetic elements of the well known cloning vector pBR322 (ATCC 37017). Such commercial vectors include, for example, pKK223-3 (Pharmacia Fine Chemicals, Uppsala, Sweden) and pGEM1 (Promega Biotec, Madison, WI, USA). These pBR322 "backbone" sections are combined with an appropriate promoter and the structural sequence to be expressed.

Following transformation of a suitable host strain and growth of the host strain to an appropriate cell density, the selected promoter is induced by appropriate means (e.g., temperature shift or chemical induction) and cells are cultured for an additional period.

Cells are typically harvested by centrifugation, disrupted by physical or chemical means, and the resulting crude extract retained for further purification.

Microbial cells employed in expression of proteins can be disrupted by any convenient method, including freeze-thaw cycling, sonication, mechanical disruption, or use of cell lysing agents, such methods are well know to those skilled in the art.

Various mammalian cell culture systems can also be employed to express recombinant protein. Examples of mammalian expression systems include the COS-7 lines of monkey kidney fibroblasts, described by Gluzman, Cell, 23:17S (1981), and other cell lines capable of expressing a compatible vector, for example, the C127, 3T3, CHO, HeLa and BHK cell lines. Mammalian expression vectors will comprise an origin of replication, a suitable promoter and enhancer, and also any necessary ribosome binding sites, polyadenylation site, splice donor and acceptor sites, transcriptional termination sequences, and 5' flanking nontranscribed sequences. DNA sequences derived from the SV40 splice, and polyadenylation sites may be used to provide the required nontranscribed genetic elements.

The polypeptides can be recovered and purified from recombinant cell cultures by methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Protein refolding steps can be used, as necessary, in completing configuration of the mature protein. Finally, high performance liquid chromatography (HPLC) can be employed for final purification steps.

The polypeptides of the present invention may be a naturally purified product, or a product of chemical synthetic procedures, or produced by recombinant techniques from a prokaryotic or eukaryotic host (for example, by bacterial, yeast, higher plant, insect and mammalian cells in culture). Depending upon the host employed in a recombinant production procedure, the polypeptides of the present invention may be glycosylated or may be non-glycosylated. Polypeptides of the invention may also include an initial methionine amino acid residue.

The polynucleotides and polypeptides of the present invention may be employed as research reagents and materials for discovery of treatments and diagnostics to human disease.

The polypeptide of the present invention may be employed to inhibit bone marrow stem cell colony formation as adjunct protective treatment during cancer chemotherapy. The Ckβ-13 polypeptide may inhibit the proliferation and differentiation of hematopoietic cells such as bone marrow stem cells. The inhibitor effect on the population of committed progenitor cells, (for example granulocytes, and macrophage/monocytes) may be employed therapeutically to inhibit proliferation of leukemic cells.

The polypeptides of the present invention may also be employed to inhibit epidermal keratinocyte proliferation for treatment of psoriasis, which is characterized by keratinocyte hyper-proliferation, since Langerhans cells in skin have been found to produce chemokines.

The polypeptides of the present invention may also be employed to treat solid tumors, for example, Karposi sarcoma, by stimulating the invasion and activation of host defense cells, e.g., cytotoxic T cells and macrophages via chemotaxis, and by inhibiting the angiogenesis of tumors.

They may also be employed to enhance host defenses against resistant chronic and acute infections, for example, mycobacterial infections via the attraction and activation of microbicidal leukocytes.

The the polypeptides of the present invention may also be employed to inhibit T cell proliferation by the inhibition of IL-2 biosynthesis for the treatment of T-cell mediated auto-immune diseases and lymphocytic leukemias.

Ckβ-13 may also be employed to stimulate wound healing and prevent scarring during wound healing, both via the recruitment of debris clearing and connective tissue promoting inflammatory cells and also via its control of excessive TGFβ-mediated fibrosis. In this same manner, Ckβ-13 may also be employed to treat other fibrotic disorders, including liver cirrhosis, osteoarthritis and pulmonary fibrosis.

The polypeptides of the present invention also increase the presence of eosinophils which have the distinctive function of killing the larvae of parasites that invade tissues, as in schistosomiasis, trichinosis and ascariasis.

They may also be employed to regulate hematopoiesis, by regulating the activation and differentiation of various hematopoietic progenitor cells, for example, to release mature leukocytes from the bone marrow following chemotherapy.

The polypeptide of the present invention may also be employed to target unwanted cells, such as in the treatment of cancer, for apoptosis.

The polynucleotides and polypeptides encoded by such polynucleotides may also be utilized for *in vitro* purposes related to scientific research, synthesis of DNA and manufacture of DNA vectors and for designing therapeutics and diagnostics for the treatment of human disease.

The polypeptide may also be used to mobilize bone marrow stem cells to peripheral blood, which allows easy isolation of stem cells. The isolated stem cells may be employed for bone marrow colonization after high dose chemotherapy.

This invention is also related to the use of the Ckβ-13 gene as part of a diagnostic assay for detecting diseases or susceptibility to diseases related to the presence of mutations in the nucleic acid sequences encoding a the polypeptide of the present invention. Such diseases are related to under-expression of the human chemokine polypeptides, for example, tumors and cancers.

Individuals carrying mutations in a gene of the present invention may be detected at the DNA level by a variety of techniques. Nucleic acids for diagnosis may be obtained from a patient's cells, such as from blood, urine, saliva, tissue biopsy and autopsy material. The genomic DNA may be used directly for detection or may be amplified enzymatically by using PCR (Saiki *et al*., Nature, 324:163-166 (1986)) prior to analysis. RNA or cDNA may also be used for the same purpose. As an example, PCR primers complementary to the nucleic acid encoding Ckβ-13 can be used to identify and analyze Ckβ-13 mutations. For example, deletions and insertions can be detected by a change in size of the amplified product in comparison to the normal genotype. Point mutations can be identified by hybridizing amplified DNA to radiolabeled Ckβ-13 RNA or alternatively, radiolabeled Ckβ-13 antisense DNA sequences. Perfectly matched sequences can be distinguished from mismatched duplexes by RNase A digestion or by differences in melting temperatures.

Genetic testing based on DNA sequence differences may be achieved by detection of alteration in electrophoretic mobility of DNA fragments in gels with or without denaturing agents. Small sequence deletions and insertions can be visualized by high resolution gel electrophoresis. DNA fragments of different sequences may be distinguished on denaturing formamide gradient gels in which the mobilities of different DNA fragments are retarded in the gel at different positions according to their specific melting or partial melting temperatures (see, e.g., Myers *et al*., Science, 230:1242 (1985)).

Sequence changes at specific locations may also be revealed by nuclease protection assays, such as RNase and S1 protection or the chemical cleavage method (e.g., Cotton *et al*., PNAS, USA, 85:4397-4401 (1985)).

Thus, the detection of a specific DNA sequence may be achieved by methods such as hybridization, RNase protection, chemical cleavage, direct DNA sequencing or the use of restriction enzymes, (e.g., Restriction Fragment Length Polymorphisms (RFLP)) and Southern blotting of genomic DNA.

In addition to more conventional gel-electrophoresis and DNA sequencing, mutations can also be detected by *in situ* analysis.

The present invention also relates to a diagnostic assay for detecting altered levels of the polypeptide of the present invention in various tissues since an over-expression of the polypeptide compared to normal control tissue samples may detect the presence of a disease or susceptibility to a disease, for example, a tumor. Assays used to detect levels of a the polypeptide of the present invention in a sample derived from a host are well-known to those of skill in the art and include radioimmunoassays, competitive-binding assays, Western Blot analysis, BLISA assays and "sandwich" assay. An BLISA assay (Coligan, et al., Current Protocols in Immunology, 1(2), Chapter 6, (1991)) initially comprises preparing an antibody specific to a Ckβ-13 antigen, preferably a monoclonal antibody. In addition a reporter antibody is prepared against the monoclonal antibody. To the reporter antibody is attached a detectable reagent such as radioactivity, fluorescence or, in this example, a horseradish peroxidase enzyme. A sample is removed from a host and incubated on a solid support, e.g. a polystyrene dish, that binds the proteins in the sample. Any free protein binding sites on the dish are then covered by incubating with a non-specific protein like BSA. Next, the monoclonal antibodv is incubated in the dish during which time the monoclonal antibodies attach to any Ckβ-13 protein attached to the polystyrene dish. All unbound monoclonal antibody is washed out with buffer. The reporter antibody linked to horseradish peroxidase is now placed in the dish resulting in binding of the reporter antibody to any monoclonal antibody bound to the Ckβ-13 polypeptide. Unattached reporter antibody is then washed out. Peroxidase substrates are then added to the dish and the amount of color developed in a given time period is a measurement of the amount of Ckβ-13 protein present in a given volume of patient sample when compared against a standard curve.

A competition assay may be employed wherein antibodies specific to the Ckβ-13 polypeptide are attached to a solid support and labeled Ckβ-13 polypeptide and a sample derived from the host are passed over the solid support and the amount of label detected, for example by liquid scintillation chromatography, can be correlated to a quantity of Ckβ-13 polypeptide in the sample.

A "sandwich" assay is similar to an BLISA assay. In a "sandwich" assay the Ckβ-13 polypeptide is passed over a solid support and binds to antibody attached to a solid support. A second antibody is then bound to the Ckβ-13 polypeptide. A third antibody which is labeled and specific to the second antibody is then passed over the solid support and binds to the second antibody and an amount can then be quantified.

This invention provides a method for identification of the receptors for the polypeptide of the present invention. The gene encoding the receptor can be identified by numerous methods known to those of skill in the art, for example, ligand panning and FACS sorting (Coligan, et al., Current Protocols in Immun., 1(2), Chapter 5, (1991)). Preferably, expression cloning is employed wherein polyadenylated RNA is prepared from a cell responsive to the polypeptides, and a cDNA library created from this RNA is divided into pools and used to transfect COS cells or other cells that are not responsive to the polypeptides. Transfected cells which are grown on glass slides are exposed to the labeled polypeptides. The polypeptides can be labeled by a variety of means including iodination or inclusion of a recognition site for a site-specific protein kinase. Following fixation and incubation, the slides are subjected to autoradiographic analysis. Positive pools are identified and sub-pools are prepared and retransfected using an iterative sub-pooling and rescreening process, eventually yielding a single clone(s) that encodes the putative receptor.

As an alternative approach for receptor identification, the labeled polypeptides can be photoaffinity linked with cell membrane or extract preparations that express the receptor molecule. Cross-linked material is resolved by PAGE analysis and exposed to X-ray film. The labeled complex containing the receptors of the polypeptides can be excised, resolved into peptide fragments, and subjected to protein microsequencing. The amino acid sequence obtained from microsequencing would be used to design a set of degenerate oligonucleotide probes to screen a cDNA library to identify the genes encoding the putative receptors.

This invention provides a method of screening compounds to identify agonists and antagonists to the polypeptide of the present invention. An agonist is a compound which binds to and activates a receptor to the polypeptide of the present invention, while antagonists bind to and inhibit such receptors or simply compete with Ckβ-13 for such receptors. Chemotaxis may be assayed by placing cells, which are chemoattracted by the polypeptide of the present invention, on top of a filter with pores of sufficient diameter to admit the cells (about 5 µm). Solutions of potential agonists are placed in the bottom of the chamber with an appropriate control medium in the upper compartment, and thus a concentration gradient of the agonist is measured by counting cells that migrate into or through the porous membrane over time.

When assaying for antagonists, the polypeptide of the present invention is placed in the bottom chamber and the potential antagonist is added to determine if chemotaxis of the cells is prevented.

Alternatively, a mammalian cell or membrane preparation expressing the receptors of the polypeptides would be incubated with a labeled polypeptide of the present invention, eg. radioactivity, in the presence of the compound. The ability of the compound to block this interaction could then be measured.

Examples of potential antagonists to the polypeptide of the present invention include antibodies, or in some cases, oligonucleotides, which bind to the polypeptides. Another example of a potential antagonist is a negative dominant mutant of the polypeptides. Negative dominant mutants are polypeptides which bind to the receptor of the wild-type polypeptide, but fail to retain biological activity.

Antisense constructs prepared using antisense technology are also potential antagonists. Antisense technology can be used to control gene expression through triple-helix formation or antisense DNA or RNA, both of which methods are based on binding of a polynucleotide to DNA or RNA. For example, the 5' coding portion of the polynucleotide sequence, which encodes for the mature polypeptides of the present invention, is used to design an antisense RNA oligonucleotide of from about 10 to 40 base pairs in length. A DNA oligonucleotide is designed to be complementary to a region of the gene involved in transcription (triple- helix, see Lee et al., Nucl. Acids Res., 6:3073 (1979); Cooney et al, Science, 241:456 (1988); and Dervan et al., Science, 251: 1360 (1991)), thereby preventing transcription and the production of the polypeptide of the present invention. The antisense RNA oligonucleotide hybridizes to the mRNA *in vivo* and blocks translation of the mRNA molecule into the polypeptides (antisense - Okano, J. Neurochem., 56:560 (1991); Oligodeoxynucleotides as Antisense Inhibitors of Gene Expression, CRC Press, Boca Raton, FL (1988)). The oligonucleotides described above can also be delivered to cells such that the antisense RNA or DNA may be expressed *in vivo* to inhibit production of the polypeptide of the present invention.

Another potential antagonist is a peptide derivative of the polypeptides which is a naturally or synthetically modified analog of the polypeptide that have lost biological function yet still recognizes and binds to the receptors of the polypeptide to thereby effectively block the receptors. Examples of peptide derivatives include, but are not limited to, small peptides or peptide-like molecules.

The antagonists may be employed to inhibit the chemotaxis and activation of macrophages and their precursors, and of neutrophils, basophils, B lymphocytes and some T cell subsets, e.g., activated and CD8 cytotoxic T cells and natural killer cells, in certain auto-immune and chronic inflammatory and infective diseases. Examples of auto-immune diseases include multiple sclerosis, and insulin-dependent diabetes.

The antagonists may also be employed to treat infectious diseases including silicosis, sarcoidosis, idiopathic pulmonary fibrosis by preventing the recruitment and activation of mononuclear phagocytes. They may also be employed to treat idiopathic hyper-eosinophilic syndrome by preventing eosinophil production and migration. Bndotoxic shock may also be treated by the antagonists by preventing the migration of macrophages and their production of the polypeptide of the present invention.

The antagonists may also be employed for treating atherosclerosis, by preventing monocyte infiltration in the artery wall.

The antagonists may also be employed to treat histamine-mediated allergic reactions and immunological disorders including late phase allergic reactions, chronic urticaria, and atopic dermatitis by inhibiting chemokine-induced mast cell and basophil degranulation and release of histamine. IgE-mediated allergic reactions such as allergic asthma, rhinitis, and eczema may also be treated.

The antagonists may also be employed to treat chronic and acute inflammation by preventing the attraction of monocytes to a wound area. They may also be employed to regulate normal pulmonary macrophage populations, since chronic and acute inflammatory pulmonary diseases are associated with sequestration of mononuclear phagocytes in the lung.

Antagonists may also be employed to treat rheumatoid arthritis by preventing the attraction of monocytes into synovial fluid in the joints of patients. Monocyte influx and activation plays a significant role in the pathogenesis of both degenerative and inflammatory arthropathies.

The antagonists may be employed to interfere with the deleterious cascades attributed primarily to IL-1 and TNF which prevents the biosynthesis of other inflammatory cytokines. In this way, the antagonists may be employed to prevent inflammation. The antagonists may also be employed to inhibit prostaglandin-independent fever induced by chemokines.

The antagonists may also be employed to treat cases of bone marrow failure, for example, aplastic anemia and myelodysplastic syndrome.

The antagonists may also be employed to treat asthma and allergy by preventing eosinophil accumulation in the lung. The antagonists may also be employed to treat subepithelial basement membrane fibrosis which is a prominent feature of the asthmatic lung.

The antagonists may be employed in a composition with a pharmaceutically acceptable carrier, e.g., as hereinafter described.

The polypeptide of the present invention and agonists and antagonists may be employed in combination with a suitable pharmaceutical carrier. Such compositions comprise a therapeutically effective amount of the polypeptide, and a pharmaceutically acceptable carrier or excipient. Such a carrier includes but is not limited to saline, buffered saline, dextrose, water, glycerol, ethanol, and combinations thereof. The formulation should suit the mode of administration.

The invention also provides a pharmaceutical pack or kit comprising one or more containers filled with one or more of the ingredients of the pharmaceutical compositions of the invention. Associated with such container(s) can be a notice in the form prescribed by a governmental agency regulating the manufacture, use or sale of pharmaceuticals or biological products, which notice reflects approval by the agency of manufacture, use or sale for human administration. In addition, the polypeptides and agonists and antagonists may be employed in conjunction with other therapeutic compounds.

The pharmaceutical compositions may be administered in a convenient manner such as by the topical, intravenous, intraperitoneal, intramuscular, intratumor, subcutaneous, intranasal or intradermal routes. The pharmaceutical compositions are administered in an amount which is effective for treating and/or prophylaxis of the specific indication. In general, the polypeptides will be administered in an amount of at least about 10 µg/kg body weight and in most cases they will be administered in an amount not in excess of about 8 mg/Kg body weight per day. In most cases, the dosage is from about 10 µg/kg to about 1 mg/kg body weight daily, taking into account the routes of administration, symptoms, etc.

The polypeptide of the present invention, and agonists or antagonists which are polypeptides, may be employed in accordance with the present invention by expression of such polypeptides *in vivo*, which is often referred to as "gene therapy."

Thus, for example, cells from a patient may be engineered with a polynucleotide (DNA or RNA) encoding a polypeptide *ex vivo*, with the engineered cells then being provided to a patient to be treated with the polypeptide. Such methods are well-known in the art. For example, cells may be engineered by procedures known in the art by use of a retroviral particle containing RNA encoding a polypeptide of the present invention.

Similarly, cells may be engineered *in vivo* for expression of a polypeptide *in vivo* by, for example, procedures known in the art. As known in the art, a producer cell for producing a retroviral particle containing RNA encoding the polypeptide of the present invention may be administered to a patient for engineering cells *in vivo* and expression of the polypeptide *in vivo*. These and other methods for administering a polypeptide of the present invention by such method should be apparent to those skilled in the art from the teachings of the present invention. For example, the expression vehicle for engineering cells may be other than a retrovirus, for example, an adenovirus which may be used to engineer cells in vivo after combination with a suitable delivery vehicle.

Retroviruses from which the retroviral plasmid vectors hereinabove mentioned may be derived include, but are not limited to, Moloney Murine Leukemia Virus, spleen necrosis virus, retroviruses such as Rous Sarcoma Virus, Harvey Sarcoma Virus, avian leukosis virus, gibbon ape leukemia virus, human immunodeficiency virus, adenovirus, Myeloproliferative Sarcoma Virus, and mammary tumor virus. In one embodiment, the retroviral plasmid vector is derived from Moloney Murine Leukemia Virus.

The vector includes one or more promoters. Suitable promoters which may be employed include, but are not limited to, the retroviral LTR; the SV40 promoter; and the human cytomegalovirus (CMV) promoter described in Miller, et al., Biotechniques, Vol. 7, No. 9, 980-990 (1989), or any other promoter (e.g., cellular promoters such as eukaryotic cellular promoters including, but not limited to, the histone, pol III, and β-actin promoters). Other viral promoters which may be employed include, but are not limited to, adenovirus promoters, thymidine kinase (TK) promoters, and B19 parvovirus promoters. The selection of a suitable promoter will be apparent to those skilled in the art from the teachings contained herein.

The nucleic acid sequence encoding the polypeptide of the present invention is under the control of a suitable promoter. Suitable promoters which may be employed include, but are not limited to, adenoviral promoters, such as the adenoviral major late promoter; or hetorologous promoters, such as the cytomegalovirus (CMV) promoter; the respiratory syncytial virus (RSV) promoter; inducible promoters, such as the MMT promoter, the metallothionein promoter; heat shock promoters; the albumin promoter; the ApoAI promoter; human globin promoters; viral thymidine kinase promoters, such as the Herpes Simplex thymidine kinase promoter; retroviral LTRs (including the modified retroviral LTRs hereinabove described); the β-actin promoter; and human growth hormone promoters. The promoter also may be the native promoter which controls the genes encoding the polypeptides.

The retroviral plasmid vector is employed to transduce packaging cell lines to form producer cell lines. Examples of packaging cells which may be transfected include, but are not limited to, the PE501, PA317, ψ-2, ψ-AM, PA12, T19-14X, VT-19-17-H2, ψCRE, ψCRIP, GP+B-86, GP+envAm12, and DAN cell lines as described in Miller, Human Gene Therapy, Vol. 1, pgs. 5-14 (1990).

The vector may transduce the packaging cells through any means known in the art. Such means include, but are not limited to, electroporation, the use of liposomes, and CaPO₄ precipitation. In one alternative, the retroviral plasmid vector may be encapsulated into a liposome, or coupled to a lipid, and then administered to a host.

The producer cell line generates infectious retroviral vector particles which include the nucleic acid sequence(s) encoding the polypeptides. Such retroviral vector particles then may be employed, to transduce eukaryotic cells, either *in vitro* or *in vivo*. The transduced eukaryotic cells will express the nucleic acid sequence(s) encoding the polypeptide. Bukaryotic cells which may be transduced include, but are not limited to, embryonic stem cells, embryonic carcinoma cells, as well as hematopoietic stem cells, hepatocytes, fibroblasts, myoblasts, keratinocytes, endothelial cells, and bronchial epithelial cells.

The sequences of the present invention are also valuable for chromosome identification. The sequence is specifically targeted to and can hybridize with a particular location on an individual human chromosome. Moreover, there is a current need for identifying particular sites on the chromosome. Few chromosome marking reagents based on actual sequence data (repeat polymorphisms) are presently available for marking chromosomal location. The mapping of DNAs to chromosomes according to the present invention is an important first step in correlating those sequences with genes associated with disease.

Briefly, sequences can be mapped to chromosomes by preparing PCR primers (preferably 15-25 bp) from the cDNA. Computer analysis of the 3' untranslated region is used to rapidly select primers that do not span more than one exon in the genomic DNA, thus complicating the amplification process. These primers are then used for PCR screening of somatic cell hybrids containing individual human chromosomes. Only those hybrids containing the human gene corresponding to the primer will yield an amplified fragment.

PCR mapping of somatic cell hybrids is a rapid procedure for assigning a particular DNA to a particular chromosome. Using the present invention with the same oligonucleotide primers, sublocalization can be achieved with panels of fragments from specific chromosomes or pools of large genomic clone(s) in an analogous manner. Other mapping strategies that can similarly be used to map to its chromosome include *in situ* hybridization, prescreening with labeled flow-sorted chromosomes and preselection by hybridization to construct chromosome specific-cDNA libraries.

Fluorescence *in situ* hybridization (FISH) of a cDNA clone(s) to a metaphase chromosomal spread can be used to provide a precise chromosomal location in one step. This technique can be used with cDNA as short as 500 or 600 bases. For a review of this technique, see Verma et al., Human Chromosomes: a Manual of Basic Techniques, Pergamon Press, New York (1988).

Once a sequence has been mapped to a precise chromosomal location, the physical position of the sequence on the chromosome can be correlated with genetic map data. Such data are found, for example, in V. McKusick, Mendelian Inheritance in Man (available on line through Johns Hopkins University Welch Medical Library). The relationship between genes and diseases that have been mapped to the same chromosomal region are then identified through linkage analysis (coinheritance of physically adjacent genes).

Next, it is necessary to determine the differences in the cDNA or genomic sequence between affected and unaffected individuals. If a mutation is observed in some or all of the affected individuals but not in any normal individuals, then the mutation is likely to be the causative agent of the disease.

With current resolution of physical mapping and genetic mapping techniques, a cDNA precisely localized to a chromosomal region associated with the disease could be one of between 50 and 500 potential causative genes. (This assumes 1 megabase mapping resolution and one gene per 20 kb).

The polypeptides, their fragments or other derivatives, or analogs thereof, or cells expressing them can be used as an immunogen to produce antibodies thereto. These antibodies can be, for example, polyclonal or monoclonal antibodies. The present invention also includes chimeric, single chain, and humanized antibodies, as well as Fab fragments, or the product of an Fab expression library. Various procedures known in the art may be used for the production of such antibodies and fragments.

Antibodies generated against the polypeptides corresponding to a sequence of the present invention can be obtained by direct injection of the polypeptides into an animal or by administering the polypeptides to an animal, preferably a nonhuman. The antibody so obtained will then bind the polypeptides itself. In this manner, even a sequence encoding only a fragment of the polypeptides can be used to generate antibodies binding the whole native polypeptides. Such antibodies can then be used to isolate the polypeptide from tissue expressing that polypeptide.

For preparation of monoclonal antibodies, any technique which provides antibodies produced by continuous cell line cultures can be used. Examples include the hybridoma technique (Kohler and Milstein, 1975, Nature, 256:495-497), the trioma technique, the human B-cell hybridoma technique (Kozbor et al., 1983, Immunology Today 4:72), and the EBV-hybridoma technique to produce human monoclonal antibodies (Cole, et al., 1985, in Monoclonal Antibodies and Cancer Therapy, Alan R. Liss, Inc., pp. 77-96).

Techniques described for the production of single chain antibodies (U.S. Patent 4,946,778) can be adapted to produce single chain antibodies to immunogenic polypeptide products of this invention. Also, transgenic mice may be used to express humanized antibodies to immunogenic polypeptide products of this invention.

The present invention will be further described with reference to the following examples; however, it is to be understood that the present invention is not limited to such examples. All parts or amounts, unless otherwise specified, are by weight.

In order to facilitate understanding of the following examples certain frequently occurring methods and/or terms will be described.

"Plasmids" are designated by a lower case p preceded and/or followed by capital letters and/or numbers. The starting plasmids herein are either commercially available, publicly available on an unrestricted basis, or can be constructed from available plasmids in accord with published procedures. In addition, equivalent plasmids to those described are known in the art and will be apparent to the ordinarily skilled artisan.

"Digestion" of DNA refers to catalytic cleavage of the DNA with a restriction enzyme that acts only at certain sequences in the DNA. The various restriction enzymes used herein are commercially available and their reaction conditions, cofactors and other requirements were used as would be known to the ordinarily skilled artisan. For analytical purposes, typically 1 µg of plasmid or DNA fragment is used with about 2 units of enzyme in about 20 µl of buffer solution. For the purpose of isolating DNA fragments for plasmid construction, typically 5 to 50 µg of DNA are digested with 20 to 250 units of enzyme in a larger volume. Appropriate buffers and substrate amounts for particular restriction enzymes are specified by the manufacturer. Incubation times of about 1 hour at 37°C are ordinarily used, but may vary in accordance with the supplier's instructions. After digestion the reaction is electrophoresed directly on a polyacrylamide gel to isolate the desired fragment.

Size separation of the cleaved fragments is performed using 8 percent polyacrylamide gel described by Goeddel, D. *et al*., Nucleic Acids Res., 8:4057 (1980).

"Oligonucleotides" refers to either a single stranded polydeoxynucleotide or two complementary polydeoxynucleotide strands which may be chemically synthesized. Such synthetic oligonucleotides have no 5' phosphate and thus will not ligate to another oligonucleotide without adding a phosphate with an ATP in the presence of a kinase. A synthetic oligonucleotide will ligate to a fragment that has not been dephosphorylated.

"Ligation" refers to the process of forming phosphodiester bonds between two double stranded nucleic acid fragments (Maniatis, T., et al., Id., p. 146). Unless otherwise provided, ligation may be accomplished using known buffers and conditions with 10 units to T4 DNA ligase ("ligase") per 0.5 µg of approximately equimolar amounts of the DNA fragments to be ligated.

Unless otherwise stated, transformation was performed as described in the method of Graham, P. and Van der Eb, A., Virology, 52:456-457 (1973).

### Example 1

### Bacterial Expression and Purification of Ckβ-13

The DNA sequence encoding for Ckβ-13, ATCC # 97113, is initially amplified using PCR oligonucleotide primers corresponding to the 5' and 3' end sequences of the processed Ckβ-13 nucleic acid sequence (minus the putative signal peptide sequence). Additional nucleotides corresponding to the Ckβ-13 gene are added to the 5' and 3' end sequences respectively. The 5' oligonucleotide primer has the sequence 5' CCCGCATGCCCAACATGGAAGACAG 3' (SEQ ID NO:3) contains a SphI restriction enzyme site (bold) followed by 16 nucleotides of Ckβ-13 coding sequence starting from the second nucleotide of the sequence coding for the mature protein. The ATG codon is included in the SphI site. In the next codon following the ATG, the first base is from the SphI site and the remaining two bases correspond to the second and third base of the first codon (residue 29) of the putative mature protein. The 3' sequence 5' AAAGGATCCTTGGCTCAGCTTATTGAG 3' (SEQ ID NO:4) contains complementary sequences to a BamH1 site (bold) and is followed by 18 nucleotides of gene specific sequences preceding the termination codon. The restriction enzyme sites correspond to the restriction enzyme sites on the bacterial expression vector pQE-9 (Qiagen, Inc. Chatsworth, CA). pQE-9 encodes antibiotic resistance (Amp'), a bacterial origin of replication (ori), an IPTG-regulatable promoter operator (P/O), a ribosome binding site (RBS), a 6-His tag and restriction enzyme sites. pQB-9 is then digested with SphI and BamH1. The amplified sequences are ligated into pQB-9 and are inserted in frame with the sequence encoding for the histidine tag and the RBS. The ligation mixture is then used to transform the E. coli strain M15/rep 4 (Qiagen, Inc.) by the procedure described in Sambrook, J. et al., Molecular Cloning: A Laboratory Manual, Cold Spring Laboratory Press, (1989). M15/rep4 contains multiple copies of the plasmid pREP4, which expresses the lacI repressor and also confers kanamycin resistance (Kan'). Transformants are identified by their ability to grow on LB plates and ampicillin/kanamycin resistant colonies are selected. Plasmid DNA is isolated and confirmed by restriction analysis. Clone(s) containing the desired constructs are grown overnight (O/N) in liquid culture in LB media supplemented with both Amp (100 ug/ml) and Kan (25 ug/ml). The O/N culture is used to inoculate a large culture at a ratio of 1:100 to 1:250. The cells are grown to an optical density 600 (O.D.⁶⁰⁰) of between 0.4 and 0.6. IPTG ("Isopropyl-B-D-thiogalacto pyranoside") is then added to a final concentration of 1 mM. IPTG induces by inactivating the lacI repressor, clearing the P/O leading to increased gene expression. Cells are grown an extra 3 to 4 hours. Cells are then harvested by centrifugation. The cell pellet is solubilized in the chaotropic agent 6 Molar Guanidine HCl pH 5.0. After clarification, solubilized Ckβ-13 is purified from this solution by chromatography on a Nickel-Chelate column under conditions that allow for tight binding by proteins containing the 6-His tag (Hochuli, B. et al., J. Chromatography 411:177-184 (1984)). Ckβ-13 ( >98% pure) is eluted from the column in 6M guanidine HCl. Protein renaturation out of GnHCl can be accomplished by several protocols (Jaenicke, R. and Rudolph, R., Protein Structure - A Practical Approach, IRL Press, New York (1990)). Initially, step dialysis is utilized to remove the GnHCL. Alternatively, the purified protein isolated from the Ni-chelate column can be bound to a second column over which a decreasing linear GnHCL gradient is run. The protein is allowed to renature while bound to the column and is subsequently eluted with a buffer containing 250 mM Imidazole, 150 mM NaCl, 25 mM Tris-HCl pH 7.5 and 10% Glycerol. Finally, soluble protein is dialyzed against a storage buffer containing 5 mM Ammonium Bicarbonate.

### Example 2

### Expression of Recombinant Ckβ-13 in COS cells

The expression of plasmid, Ckβ-13 HA is derived from a vector pcDNAI/Amp (Invitrogen) containing: 1) SV40 origin of replication, 2) ampicillin resistance gene, 3) E.coli replication origin, 4) CMV promoter followed by a polylinker region, a SV40 intron and polyadenylation site. A DNA fragment encoding the entire Ckβ-13 precursor and a HA tag fused in frame to its 3' end is cloned into the polylinker region of the vector, therefore, the recombinant protein expression is directed under the CMV promoter. The HA tag correspond to an epitope derived from the influenza hemagglutinin protein as previously described (I. Wilson, H. Niman, R. Heighten, A Cherenson, M. Connolly, and R. Lerner, 1984, Cell 37, 767). The infusion of HA tag to the target protein allows easy detection of the recombinant protein with an antibody that recognizes the HA epitope.

The plasmid construction strategy is described as follows:

The DNA sequence encoding for Ckβ-13, ATCC # 97113, is constructed by PCR using two primers: the 5' primer 5' AAA AAGCTTAACATAGGCTCGCCTACAGACT 3' (SEQ ID NO:5) contains a HindIII site followed by 18 nucleotides of Ckβ-13 coding sequence starting from the minus 3 position relative to initiation codon; the 3' sequence 5' CGCTCTAGATTAAGCGT AGTCTGGGACGTCGTATGGGTATTGGCTCAGCTTATTGAGAAT 3' (SEQ ID NO:6) contains complementary sequences to an XbaI site, translation stop codon (underlined), HA tag and the last 21 nucleotides of the Ckβ-13 coding sequence (not including the stop codon). Therefore, the PCR product contains a HindIII site, Ckβ-13 coding sequence followed by HA tag fused in frame, a translation termination stop codon next to the HA tag, and an XbaI site. The PCR amplified DNA fragment and the vector, pcDNA3/Amp, are digested with HindIII and XbaI restriction enzyme and ligated. The ligation mixture is transformed into E. coli strain SURE (Stratagene Cloning Systems, La Jolla, CA) the transformed culture is plated on ampicillin media plates and resistant colonies are selected. Plasmid DNA is isolated from transformants and examined by restriction analysis for the presence of the correct fragment. For expression of the recombinant Ckβ-13 polypeptide, COS cells are transfected with the expression vector by DEAE-DEXTRAN method (J. Sambrook, E. Fritsch, T. Maniatis, Molecular Cloning: A Laboratory Manual, Cold Spring Laboratory Press, (1989)). The expression of the Ckβ-13 HA protein is detected by radiolabelling and immunoprecipitation method (B. Harlow, D. Lane, Antibodies: A Laboratory Manual, Cold Spring Harbor Laboratory Press, (1988)). Cells are labelled for 8 hours with ³⁵S-cysteine two days post transfection. Culture media are then collected and cells are lysed with detergent (RIPA buffer (150 mM NaCl, 1% NP-40, 0.1% SDS, 1% NP-40, 0.5% DOC, 50mM Tris, pH 7.5) (Wilson, I. et al., Id. 37:767 (1984)). Both cell lysate and culture media are precipitated with a HA specific monoclonal antibody. Proteins precipitated are analyzed by SDS-PAGE.

### Example 3

### Cloning and expression of Ckβ-13 using the baculovirus expression system

The DNA sequence encoding the full length Ckβ-13 protein, ATCC # 97113, is amplified using PCR oligonucleotide primers corresponding to the 5' and 3' sequences of the gene:

The 5' primer has the sequence 5' AAAGGATCCGCCACCATGGCTC GCCTACAGACT 3' (SEQ ID NO:7) and contains a BamHI restriction enzyme site (in bold) followed by 6 nucleotides resembling an efficient signal for the initiation of translation in eukaryotic cells (Kozak, M., J. Mol. Biol., 196:947-950 (1987) and the first 18 nucleotides of the Ckβ-13 gene (the initiation codon for translation "ATG" is underlined).

The 3' primer has the sequence 5' AAAGGTACCTCATTGG CTCAGCTTATT 3' (SBQ ID NO:8) and contains the cleavage site for the restriction endonuclease Asp718 and 18 nucleotides complementary to the 3' non-translated sequence of the Ckβ-13 gene. The amplified sequences are isolated from a 1% agarose gel using a commercially available kit ("Geneclean," BIO 101 Inc., La Jolla, Ca.). The fragment is then digested with the endonucleases BamHI and Asp718 and then purified again on a 1% agarose gel. This fragment is designated F2.

The vector pRG1 (modification of pVL941 vector, discussed below) is used for the expression of the Ckβ-13 protein using the baculovirus expression system (for review see: Summers, M.D. and Smith, G.B. 1987, A manual of methods for baculovirus vectors and insect cell culture procedures, Texas Agricultural Experimental Station Bulletin NO:1555). This expression vector contains the strong polyhedrin promoter of the Autographa californica nuclear polyhedrosis virus (AcMNPV) followed by the recognition sites for the restriction endonucleases BamHI and Asp718. The polyadenylation site of the simian virus (SV)40 is used for efficient polyadenylation. For an easy selection of recombinant viruses the beta-galactosidase gene from B.coli is inserted in the same orientation as the polyhedrin promoter followed by the polyadenylation signal of the polyhedrin gene. The polyhedrin sequences are flanked at both sides by viral sequences for the cell-mediated homologous recombination of cotransfected wild-type viral DNA. Many other baculovirus vectors could be used in place of pRG1 such as pAc373, pVL941 and pAcIM1 (Luckow, V.A. and Summers, M.D., Virology, 170:31-39).

The plasmid is digested with the restriction enzymes BamHI and Asp718 and then dephosphorylated using calf intestinal phosphatase by procedures known in the art. The DNA is then isolated from a 1% agarose gel using the commercially available kit ("Geneclean" BIO 101 Inc., La Jolla, Ca.). This vector DNA is designated V2.

Fragment F2 and the dephosphorylated plasmid V2 are ligated with T4 DNA ligase. E.coli HB101 cells are then transformed and bacteria identified that contained the plasmid (pBac-Ckβ-13) with the Ckβ-13 gene using the enzymes BamHI and Asp718. The sequence of the cloned fragment is confirmed by DNA sequencing.

5 µg of the plasmid pBac-Ckβ-13 is cotransfected with 1.0 µg of a commercially available linearized baculovirus ("BaculoGold™ baculovirus DNA", Pharmingen, San Diego, CA.) using the lipofection method (Felgner et al. Proc. Natl. Acad. Sci. USA, 84:7413-7417 (1987)).

1µg of BaculoGold™ virus DNA and 5 µg of the plasmid pBac-Ckβ-13 are mixed in a sterile well of a microtiter plate containing 50 µl of serum free Grace's medium (Life Technologies Inc., Gaithersburg, MD). Afterwards 10 µl Lipofectin plus 90 µl Grace's medium are added, mixed and incubated for 15 minutes at room temperature. Then the transfection mixture is added dropwise to the Sf9 insect cells (ATCC CRL 1711) seeded in a 35 mm tissue culture plate with 1ml Grace's medium without serum. The plate is rocked back and forth to mix the newly added solution. The plate is then incubated for 5 hours at 27°C. After 5 hours the transfection solution is removed from the plate and 1 ml of Grace's insect medium supplemented with 10% fetal calf serum is added. The plate is put back into an incubator and cultivation continued at 27°C for four days.

After four days the supernatant is collected and a plaque assay performed similar as described by Summers and Smith (supra). As a modification an agarose gel with "Blue Gal" (Life Technologies Inc., Gaithersburg) is used which allows an easy isolation of blue stained plaques. (A detailed description of a "plaque assay" can also be found in the user's guide for insect cell culture and baculovirology distributed by Life Technologies Inc., Gaithersburg, page 9-10).

Four days after the serial dilution, the viruses are added to the cells and blue stained plaques are picked with the tip of an Bppendorf pipette. The agar containing the recombinant viruses is then resuspended in an Bppendorf tube containing 200 µl of Grace's medium. The agar is removed by a brief centrifugation and the supernatant containing the recombinant baculovirus is used to infect Sf9 cells seeded in 35 mm dishes. Four days later the supernatants of these culture dishes are harvested and then stored at 4°C.

Sf9 cells are grown in Grace's medium supplemented with 10% heat-inactivated FBS. The cells are infected with the recombinant baculovirus V-Ckβ-13 at a multiplicity of infection (MOI) of 2. Six hours later the medium is removed and replaced with SF900 II medium minus methionine and cysteine (Life Technologies Inc., Gaithersburg). 42 hours later 5 µCi of ³⁵S-methionine and 5 µCi ³⁵S cysteine (Amersham) are added. The cells are further incubated for 16 hours before they are harvested by centrifugation and the labelled proteins visualized by SDS-PAGB and autoradiography.

### Example 4

### Expression via Gene Therapy

Fibroblasts are obtained from a subject by skin biopsy. The resulting tissue is placed in tissue-culture medium and separated into small pieces. Small chunks of the tissue are placed on a wet surface of a tissue culture flask, approximately ten pieces are placed in each flask. The flask is turned upside down, closed tight and left at room temperature over night. After 24 hours at room temperature, the flask is inverted and the chunks of tissue remain fixed to the bottom of the flask and fresh media (e.g., Ham's F12 media, with 10% FBS, penicillin and streptomycin, is added. This is then incubated at 37°C for approximately one week. At this time, fresh media is added and subsequently changed every several days. After an additional two weeks in culture, a monolayer of fibroblasts emerge. The monolayer is trypsinized and scaled into larger flasks.

pMV-7 (Kirschmeier, P.T. et al, DNA, 7:219-25 (1988) flanked by the long terminal repeats of the Moloney murine sarcoma virus, is digested with EcoRI and HindIII and subsequently treated with calf intestinal phosphatase. The linear vector is fractionated on agarose gel and purified, using glass beads.

The cDNA encoding a polypeptide of the present invention is amplified using PCR primers which correspond to the 5' and 3' end sequences respectively. The 5' primer containing an EcoRI site and the 3' primer further includes a HindIII site. Equal quantities of the Moloney murine sarcoma virus linear backbone and the amplified EcoRI and HindIII fragment are added together, in the presence of T4 DNA ligase. The resulting mixture is maintained under conditions appropriate for ligation of the two fragments. The ligation mixture is used to transform bacteria HB101, which are then plated onto agar-containing kanamycin for the purpose of confirming that the vector had the gene of interest properly inserted.

The amphotropic pA317 or GP+am12 packaging cells are grown in tissue culture to confluent density in Dulbecco's Modified Eagles Medium (DMEM) with 10% calf serum (CS), penicillin and streptomycin. The MSV vector containing the gene is then added to the media and the packaging cells are transduced with the vector. The packaging cells now produce infectious viral particles containing the gene (the packaging cells are now referred to as producer cells).

Fresh media is added to the transduced producer cells, and subsequently, the media is harvested from a 10 cm plate of confluent producer cells. The spent media, containing the infectious viral particles, is filtered through a millipore filter to remove detached producer cells and this media is then used to infect fibroblast cells. Media is removed from a sub-confluent plate of fibroblasts and quickly replaced with the media from the producer cells. This media is removed and replaced with fresh media. If the titer of virus is high, then virtually all fibroblasts will be infected and no selection is required. If the titer is very low, then it is necessary to use a retroviral vector that has a selectable marker, such as neo or his.

The engineered fibroblasts are then injected into the host, either alone or after having been grown to confluence on cytodex 3 microcarrier beads. The fibroblasts now produce the protein product.

Numerous modifications and variations of the present invention are possible in light of the above teachings and, therefore, within the scope of the appended claims, the invention may be practiced otherwise than as particularly described.

## Claims

1. A polynucleotide selected from the group consisting of
(a) polynucleotides encoding at least the mature form of the polypeptide having the deduced amino acid sequence as shown in SEQ ID No. 2;
(b) polynucleotides having the coding sequence as shown in SEQ ID No. 1 encoding at least the mature form of the polypeptide;
(c) polynucleotides encoding the polypeptide having the amino acid sequence of at least the mature form of the polypeptide encoded by the cDNA contained in ATCC 97113;
(d) polynucleotides having the coding sequence of the cDNA contained in ATCC 97113 encoding at least the mature form of the polypeptide;
(e) polynucleotides encoding a fragment of a polypeptide encoded by a polynucleotide of any one of (a) to (d) wherein said fragment has chemotactic activity;
(f) polynucleotides encoding a polypeptide fragment of at least 30 or at least 50 amino acid residues of a polypeptide encoded by a polynucleotide of any one of (a) to (d), wherein said fragment has chemotactic activity; and
(g) polynucleotides at least 70% identical to a polynucleotide as defined in any one of (a) to (f) and which encode a polypeptide having chemotactic activity;
or the complementary strand of such a polynucleotide.

2. The polynucleotide of claim 1 which is DNA.

3. The DNA of claim 2 which is genomic DNA.

4. The polynucleotide of claim 1 which is RNA.

5. A vector containing the polynucleotide of any one of claims 1 to 4.

6. The vector of claim 5 in which the polynucleotide is operatively linked to expression control sequences allowing expression in prokaryotic or eukaryotic host cells.

7. A host cell genetically engineered with the vector of claim 5 or 6.

8. A process for producing a polypeptide having chemotactic activity comprising: culturing the host cell of claim 7 and recovering the polypeptide encoded by said polynucleotide from the culture.

9. A process for producing cells capable of expressing a polypeptide having chemotactic activity comprising genetically engineering cells with the vector of claim 5 or 6.

10. A polypeptide having the amino acid sequence encoded by a polynucleotide of any one of claims 1 to 4 or obtainable by the process of claim 8.

11. An antibody against the polypeptide of claim 10.

12. A nucleic acid molecule which specifically hybridizes under stringent conditions to a polynucleotide of any one of claims 1 to 4.

13. An antagonist/inhibitor against the polypeptide of claim 10 wherein said antagonist/inhibitor is an antibody or antisense construct which hybridizes under stringent conditions to a transcript of a polynucleotide of any one of claims 1 to 4.

14. A pharmaceutical composition comprising the polynucleotide of any one of claims 1 to 4, the polypeptide of claim 10 or a DNA encoding and capable of expressing said polypeptide in vivo, or the antagonist of claim 13 and optionally a pharmaceutically acceptable carrier.

15. A diagnostic composition comprising the polynucleotide of any one of claims 1 to 4, the nucleic acid molecule of claim 12 or the antibody of claim 11.

16. Use of the polypeptide of claim 10 or of the polynucleotide of any one of claims 1 to 4 for the preparation of a pharmaceutical composition for the treatment of solid tumors, chronic infections, leukemia, T-cell mediated auto-immune diseases, parasitic infections, psoriasis, for the regulation of hematopoiesis, for stimulating growth factor activity, for inhibiting angiogenesis or for promoting wound healing.

17. Use of the antagonist of claim 13 for the preparation of a pharmaceutical composition for the treatment of auto-immune diseases, atherosclerosis, chronic inflammatory or infectious diseases, histamine or IgE-mediated allergic reactions, prostaglandin-independent fever, bone marrow failure, silicosis, sarcoidosis, rheumatoid arthritis, or hyper-eosinophilic syndrome.

18. An in vitro process for diagnosing a disease or a susceptibility to a disease related to an under-expression of the polypeptide of claim 10 comprising: determining a mutation in the nucleic acid sequence encoding said polypeptide.

19. A diagnostic process comprising: analyzing for the presence of the polypeptide of claim 10 in a sample derived from a host.

20. A process for identifying an agonist to the polypeptide of claim 10 comprising:
(a) combining a compound to be screened and a reaction mixture containing cells under conditions where the cells normally migrate in response to the polypeptide of claim 10; and
(b) determining the extent of migration of the cells to identify if the compound is effective as an agonist.

21. The process of claim 20 wherein the polypeptide of claim 10 is added to the combination of step (a), and the determination of the extent of migration identifies a compound effective as an antagonist.

## Patentansprüche

1. Polynucleotid ausgewählt aus der Gruppe bestehend aus
(a) Polynucleotiden, die zumindest die reife Form des Polypeptids codieren, das die abgeleitete Aminosäuresequenz wie in SEQ ID NO: 2 gezeigt aufweist;
(b) Polynucleotiden, die die codierende Sequenz wie gezeigt in SEQ ID NO: 1, die zumindest die reife Form des Polypeptids codiert, aufweisen;
(c) Polynucleotiden, die das Polypeptid codieren, das die Aminosäuresequenz von zumindest der reifen Form des Polypeptids aufweist, das durch die cDNA, die in ATCC 97113 enthalten ist, codiert wird;
(d) Polynucleotiden, die die codierende Sequenz der cDNA, die in ATCC 97113 enthalten ist, aufweisen, die zumindest die reife Form des Polypeptids codiert;
(e) Polynucleotiden, die ein Fragment eines Polypeptids codieren, das durch ein Polynucleotid nach einem der Teile (a) bis (d) codiert wird, wobei das Fragment chemotaktische Aktivität aufweist;
(f) Polynucleotiden, die ein Polypeptidfragment mit mindestens 30 oder mindestens 50 Aminosäureresten eines Polypeptids codieren, das durch ein Polynucleotid nach einem der Teile (a) bis (d) codiert wird, wobei das Fragment chemotaktische Aktivität aufweist; und
(g) Polynucleotiden, die zumindest zu 70% identisch sind mit einem Polynucleotid wie in einem der Teile (a) bis (f) definiert und die ein Polypeptid codieren, das chemotaktische Aktivität aufweist;
oder der komplementäre Strang eines solchen Polynucleotids.

2. Polynucleotid nach Anspruch 1, das DNA ist.

3. DNA nach Anspruch 2, die genomische DNA ist.

4. Polynucleotid nach Anspruch 1, das RNA ist.

5. Vektor enthaltend das Polynucleotid nach einem der Ansprüche 1 bis 4.

6. Vektor nach Anspruch 5, in dem das Polynucleotid mit Expressionskontrollsequenzen, die die Expression in prokaryontischen oder eukaryontischen Wirtszellen erlauben, funktionell verbunden ist.

7. Wirtszelle, die genetisch verändert ist mit dem Vektor nach Anspruch 5 oder 6.

8. Verfahren zur Herstellung eines Polypeptids, das chemotaktische Aktivität aufweist, umfassend: Züchten der Wirtszelle nach Anspruch 7 und Gewinnen des Polypeptids, das von dem Polynucleotid codiert wird, aus der Kultur.

9. Verfahren zur Herstellung von Zellen, die in der Lage sind, ein Polypeptid, das chemotaktische Aktivität aufweist, zu exprimieren, umfassend das genetische Verändern von Zellen mit dem Vektor nach Anspruch 5 oder 6.

10. Polypeptid, das die Aminosäuresequenz, die von einem Polynucleotid nach einem der Ansprüche 1 bis 4 codiert wird, aufweist oder durch das Verfahren nach Anspruch 8 erhältlich ist.

11. Antikörper, der gegen das Polypeptid nach Anspruch 10 gerichtet ist.

12. Nucleinsäuremolekül, das unter stringenten Bedingungen spezifisch an ein Polynucleotid nach einem der Ansprüche 1 bis 4 hybridisiert.

13. Antagonist/Inhibitor, der gegen das Polypeptid von Anspruch 10 gerichtet ist, wobei der Antagonist/Inhibitor ein Antikörper oder ein Antisense-Konstrukt ist, wobei das Antisense-Konstrukt unter stringenten Bedingungen mit einem Transkript eines Polynucleotids nach einem der Ansprüche 1 bis 4 hybridisiert.

14. Arzneimittel umfassend das Polynucleotid nach einem der Ansprüche 1 bis 4, das Polypeptid nach Anspruch 10 oder eine DNA, die das Polypeptid codiert und in der Lage ist, das Polypeptid in vivo zu exprimieren, oder einen Antagonist nach Anspruch 13 und gegebenenfalls einen pharmazeutisch wirksamen Träger.

15. Diagnostisches Mittel umfassend das Polynucleotid nach einem der Ansprüche 1 bis 4, das Nucleinsäuremolekül nach Anspruch 12 oder den Antikörper nach Anspruch 11.

16. Verwendung des Polypeptids nach Anspruch 10 oder des Polynucleotids nach einem der Ansprüche 1 bis 4 für die Herstellung eines Arzneimittels zur Behandlung von festen Tumoren, chronischen Infektionen, Leukämie, T-Zellvermittelten Autoimmunkrankheiten, parasitären Infektionen, Schuppenflechte, zur Regulation der Hämatopoese, zur Stimulierung von Wachstumsfaktoraktivität, zur Hemmung von Angiogenese oder zur Förderung von Wundheilung.

17. Verwendung des Antagonisten nach Anspruch 13 für die Herstellung eines Arzneimittels zur Behandlung von Autoimmunkrankheiten, Atherosklerose, chronischen entzündlichen oder infektiösen Krankheiten, Histamin- oder IgE-vermittelten allergischen Reaktionen, Prostaglanidin-unabhängigem Fieber, Knochenmarkinsuffizienz, Silikose, Sarkoidose, rheumatoider Arthritis oder hypereosinophilem Syndrom.

18. In vitro-Verfahren zur Diagnose einer Krankheit oder einer Anfälligkeit für eine Krankheit, die einer Unterexpression des Polypeptids nach Anspruch 10 zuzurechnen ist, umfassend: Bestimmen einer Mutation in der Nucleinsäuresequenz, die das Polypeptid codiert.

19. Diagnostisches Verfahren umfassend: Nachweisen des Vorhandenseins des Polypeptids nach Anspruch 10 in einer Probe, die einem Wirt entnommen wurde.

20. Verfahren zur Identifizierung eines Agonisten zu dem Polypeptid nach Anspruch 10 umfassend:
(a) Vereinigen einer zu durchmusternden Verbindung und eines Reaktionsgemisches, das Zellen enthält, unter Bedingungen, unter denen die Zellen als Antwort auf das Polypeptid nach Anspruch 10 normalerweise migrieren; und
(b) Bestimmen des Ausmaßes der Migration der Zellen, um festzustellen, ob die Verbindung als Agonist wirksam ist.

21. Verfahren nach Anspruch 20, wobei das Polypeptid von Anspruch 10 dem vereinten Gemisch von Schritt (a) zugesetzt wird, und die Bestimmung des Ausmaßes der Migration eine Verbindung als wirksamen Antagonisten identifiziert.

## Revendications

1. Polynucléotide choisi dans le groupe constitué par :
(a) les polynucléotides encodant au moins la forme mature du polypeptide ayant la séquence déduite en acides aminés comme montré dans SEQ ID No. 2 ;
(b) les polynucléotides ayant la séquence codante comme montré dans SEQ ID No. 1 encodant au moins la forme mature du polypeptide ;
(c) les polynucléotides encodant le polypeptide ayant la séquence en acides aminés d'au moins la forme mature du polypeptide encodé par l'ADNc contenu dans ATCC 97113 ;
(d) les polynucléotides ayant la séquence codante de l'ADNc contenu dans ATCC 97113 encodant au moins la forme mature du polypeptide ;
(e) les polynucléotides encodant un fragment d'un polypeptide encodé par un polynucléotide selon l'un quelconque de (a) à (d), dans lesquels ledit fragment possède une activité chimiotactique ;
(f) les polynucléotides encodant un fragment de polypeptide d'au moins 30 ou d'au moins 50 résidus d'acides aminés d'un polypeptide encodé par un polynucléotide selon l'un quelconque de (a) à (d), dans lesquels ledit fragment possède une activité chimiotactique ; et
(g) les polynucléotides identiques à au moins 70 % à un polynucléotide selon l'un quelconque de (a) à (f), et qui encodent un polypeptide possédant une activité chimiotactique ;
ou le brin complémentaire d'un tel polynucléotide.

2. Polynucléotide selon la revendication 1, qui est l'ADN.

3. ADN selon la revendication 2, qui est l'ADN génomique.

4. Polynucléotide selon la revendication 1, qui est l'ARN.

5. Vecteur contenant le polynucléotide selon l'une quelconque des revendications 1 à 4.

6. Vecteur selon la revendication 5, dans lequel le polynucléotide est lié de façon opératoire aux séquences de contrôle de l'expression permettant l'expression dans des cellules-hôtes procaryotes ou eucaryotes.

7. Cellule-hôte génétiquement modifiée avec le vecteur selon la revendication 5 ou 6.

8. Procédé de production d'un polypeptide possédant une activité chimiotactique, comprenant : la culture de la cellule-hôte selon la revendication 7 et la récupération du polypeptide encodé par ledit polynucléotide à partir de la culture.

9. Procédé de production de cellules capables d'exprimer un polypeptide possédant une activité chimiotactique, comprenant des cellules génétiquement modifiées avec le vecteur selon la revendication 5 ou 6.

10. Polypeptide ayant la séquence en acides aminés encodée par un polynucléotide selon l'une quelconque des revendications 1 à 4, ou pouvant être obtenu par le procédé selon la revendication 8.

11. Anticorps contre le polypeptide selon la revendication 10.

12. Molécule d'acide nucléique qui s'hybride spécifiquement dans des conditions stringentes en un polynucléotide selon l'une quelconque des revendications 1 à 4.

13. Antagoniste/inhibiteur contre le polypeptide selon la revendication 10, dans lequel ledit antagoniste/inhibiteur est un anticorps ou une construction antisens qui s'hybride dans des conditions stringentes en un produit de transcription d'un polynucléotide selon l'une quelconque des revendications 1 à 4.

14. Composition pharmaceutique comprenant le polynucléotide selon l'une quelconque des revendications 1 à 4, le polypeptide selon la revendication 10 ou un ADN encodant et capable d'exprimer ledit polypeptide in vivo, ou l'antagoniste selon la revendication 13 et facultativement un support pharmaceutiquement acceptable.

15. Composition de diagnostic comprenant le polynucléotide selon l'une quelconque des revendications 1 à 4, la molécule d'acide nucléique selon la revendication 12 ou l'anticorps selon la revendication 11.

16. Utilisation du polypeptide selon la revendication 10 ou du polynucléotide selon l'une quelconque des revendications 1 à 4, pour la préparation d'une composition pharmaceutique pour le traitement des tumeurs solides, des infections chroniques, de la leucémie, des maladies auto-immunes faisant intervenir les lymphocytes T, des infections parasites, du psoriasis, pour la régulation de l'hématopoïèse, pour stimuler l'activité du facteur de croissance, pour inhiber l'angiogenèse ou pour permettre la guérison des blessures.

17. Utilisation de l'antagoniste selon la revendication 13, pour la préparation d'une composition pharmaceutique pour le traitement des maladies auto-immunes, de l'athérosclérose, des maladies inflammatoires ou infectieuses chroniques, des réactions allergiques faisant intervenir l'histamine ou l'immunoglobuline E, de la fièvre indépendante de la prostaglandine, de l'aplasie médullaire, de la silicose, de la sarcoïdose, de l'arthrite rhumatoïde, ou du syndrome hyper-éosinophilique.

18. Procédé in vitro pour diagnostiquer une maladie ou une susceptibilité à une maladie associée à une sous-expression du polypeptide selon la revendication 10, comprenant : la détermination d'une mutation dans la séquence en acides nucléiques encodant ledit polypeptide.

19. Procédé de diagnostic comprenant : l'analyse de la présence du polypeptide selon la revendication 10 dans un échantillon dérivé d'un hôte.

20. Procédé pour identifier un agoniste du polypeptide selon la revendication 10, comprenant :
(a) la combinaison d'un composé destiné à être passé au crible et d'un mélange réactionnel contenant des cellules dans des conditions où les cellules migrent normalement en réponse au polypeptide selon la revendication 10 ; et
(b) la détermination de l'étendue de la migration des cellules pour identifier si le composé est efficace en tant qu'agoniste.

21. Procédé selon la revendication 20, dans lequel le polypeptide selon la revendication 10 est ajouté à la combinaison de l'étape (a), et la détermination de l'étendue de la migration identifie un composé efficace en tant qu'antagoniste.
